# EUROPEAN PATENT APPLICATION

(11) **EP 4 177 607 A1**
(43) Date of publication of application: **10.05.2023**
(21) Application number: 22204974.4
(22) Date of filing: 02.11.2022
(51) Int. Cl.: G01N 33/533, G01N 33/58, G01N 33/532, G01N 33/534, G01N 33/60

(54) **BRIGHT AND RELEASABLE LABELS FOR CELL STAINING BASED ON THE CONJUGATES WITH SEVERAL SITES OF FLUOROPHORE RELEASE**

(30) Priority: 04.11.2021 EP 21206350
(71) Applicant: Miltenyi Biotec B.V. & Co. KG, 51429 Bergisch Gladbach (DE)
(72) Inventor: YUSHCHENKO, Dmytro, 51429 Bergisch Gladbach (DE); REIBER, Thorge, 51429 Bergisch Gladbach (DE)
(74) Representative: Kisters, Michael Marcus

(57) **Abstract**

The invention is directed to a conjugate characterized by high brightness to enable detection of rarely expressed epitopes and release of the label from the epitope to enable downstream applications such as sequential imaging or cell sorting and with the general formula (I) Yn - P1(P2 - Xₘ)ₒ, with X: detection moiety; PI: first enzymatically degradable spacer; P2: second enzymatically degradable spacer; Y: antigen recognizing moiety and n, m, o integers between 1 and 100 with the provision that first spacer PI and second spacer P2 are not degradable by the same enzyme.

## Description

### BACKGROUND

The present invention discloses a process for cell detection by labeling the target cells with a conjugate having a detection moiety and an antigen recognizing moiety linked via two different enzymatically degradable spacers.

Cell analysis and cell separation techniques such as Fluorescence microscopy, Flow cytometry, and Cell sorting are among the key techniques for the detailed analysis and specific isolation of target cells from a biological specimen in both research and clinical applications. They rely on fluorescent labeling of epitopes by fluorescent conjugates.

There are several technological limitations related to the use of conventional fluorescent conjugates for cell labeling, among them:
- Achieving high fluorescence brightness of the conjugates to enable detection of rarely expressed epitopes.
- Release of the label from the epitope to enable downstream applications such as sequential imaging or cell sorting.

Especially for technologies based on sequential cycles of labelling-detection-elimination with high multiplexing potential to map, e.g., protein networks, elimination of the fluorescence signal is essential.

Several approaches of releasable labels were developed in last years. For example, EP3037821A1 discloses conjugates which enable specific cell labeling but wherein the fluorophore can be cleaved and washed out after the experiment enabling subsequent staining and imaging.

WO20080918100 discloses a conjugate wherein an enzyme acts as activator for a fluorescent dye, which can be released from the target cells by radiation induced cleaving of the enzyme.

However, these reagents rely on a single enzymatically digestible spacer. As all enzymatic reactions have an equilibrium, the release of the dye will never be complete and some residual fluorescence will remain with the sample.

### SUMMARY

It was therefore an object of the invention to provide staining reagents for labeling and detection of a target in or on a sample of a biological specimen and subsequent removing the detection moiety in order to provide reversible labeling and/or enable further different labeling and detection cycles which can be released to a higher degree, i.e. to reduce residual fluorescence after release.

To solve the aforementioned problems related to release of fluorescent labeling, a new approach for the design of releasable conjugates is proposed. It relies on conjugates with a general structure (I) Yn - P1(P2 - X m)o, where X: detection moiety; P1: and P2 are enzymatically degradable spacers which are not degradable by the same enzyme.

Object of the invention is a conjugate with the general formula (I) Yn - P1(P2 - X m)o, with X: detection moiety; P1: first enzymatically degradable spacer; P2: second enzymatically degradable spacer; Y: antigen recognizing moiety and n, m, o integers between 1 and 100 with the provision that first spacer P1 and second spacer P2 are not degradable by the same enzyme.

General formula (I) requires P1 to bind to one or more (1 to "o") units of P2. For sake of clarity, general formula (I) can be termed in the following as Yn - P1- (P2 - X m)o.

Such design of conjugates permits:
- high multimerization of the fluorophores avoiding their significant self-quenching that is often difficult to achieve by using one polymer backbone;
- Efficient release of the dyes from the binder due to the double digestion of the spacers between the binder and the dyes, that is often challenging when one digestible spacer is used.

Another object of the invention is a method for detecting a target moiety in a sample of biological specimens by:
a) providing at least one conjugate with the general formula (I) Yₙ - P1(P2 - Xₘ)ₒ, with X: detection moiety; P1: first enzymatically degradable spacer; P2: second enzymatically degradable spacer; and Y: antigen recognizing moiety and n, m, o are integers between 1 and 100
b) contacting the sample of biological specimens with at least one conjugate, thereby labeling the target moiety recognized by the antigen recognizing moiety Y
c) detecting the target moiety labeled with the conjugate with the detecting moiety X and
d) enzymatically degrading first spacer P1 and/or second P2 by providing a first enzyme capable of degrading first spacer P1 and/or a second enzyme capable of degrading second spacer enzyme P2, thereby cleaving the detection moiety X from the conjugate wherein the first enzyme is not capable of degrading second spacer P2 and the second enzyme is not capable of degrading first spacer P1.

The coupling of spacer P1 and P2 can be performed using standard coupling chemistry. E.g. coupling of oligonucleotides as P2 to dextran as P1 will be realized by thiol-maleimide chemistry. At the same time, the antigen recognizing moiety e.g. an antibody or antibody fragment, can be attached to the dextran as P1 by using orthogonal reaction. These conjugates can be used as templates for desired modifications. E.g, the respective functionalization of oligonucleotides can be performed via covalent modification with a fluorophore as detection moiety X or by hybridization with functionalized complimentary oligonucleotides coupled to a detection moiety X. The resulting conjugates contain two sites for a release mechanism, e.g. dextran and oligonucleotides for dextranase and DNase respectively that ensures efficient removal of staining from the labelled antigen.

With the conjugates and the method of the invention, a more complete "enzymatic destaining" of the detection moieties can be achieved, thus reducing residual staining and hence residual background signal in sequential imaging.

### BRIEF DESCRIPTION OF THE DRAWINGS

Fig. 1 shows Staining and destaining with conjugates having one site of detection moiety release, as well as limitations of this approach

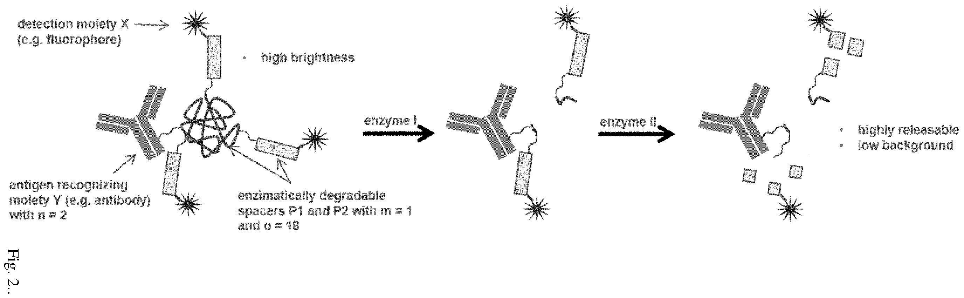

Fig. 2 shows exemplary conjugate according to the claims that can be degraded by different enzymes in two ways. The first enzyme cleaves spacer P1, the other one cleaves spacer P2

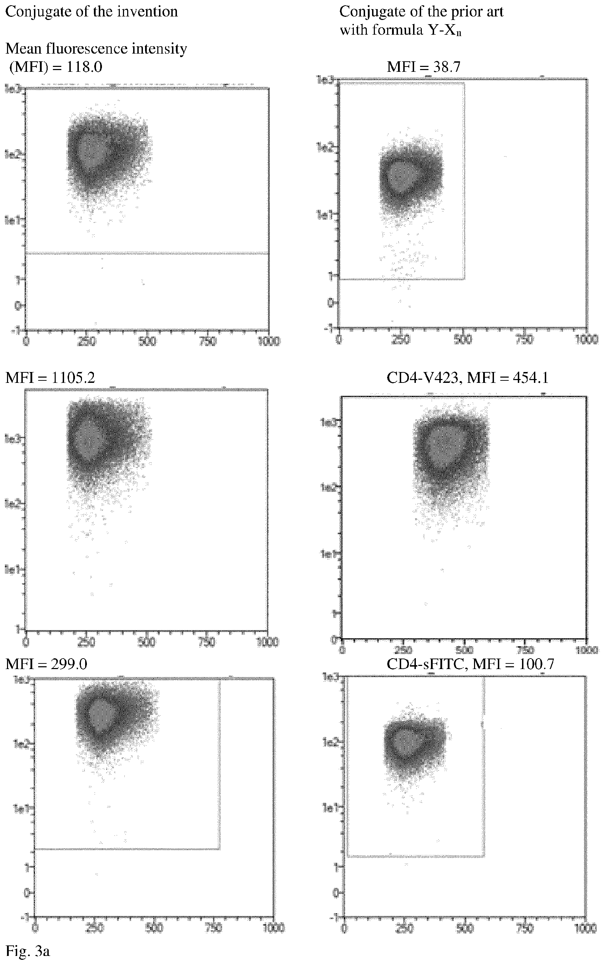

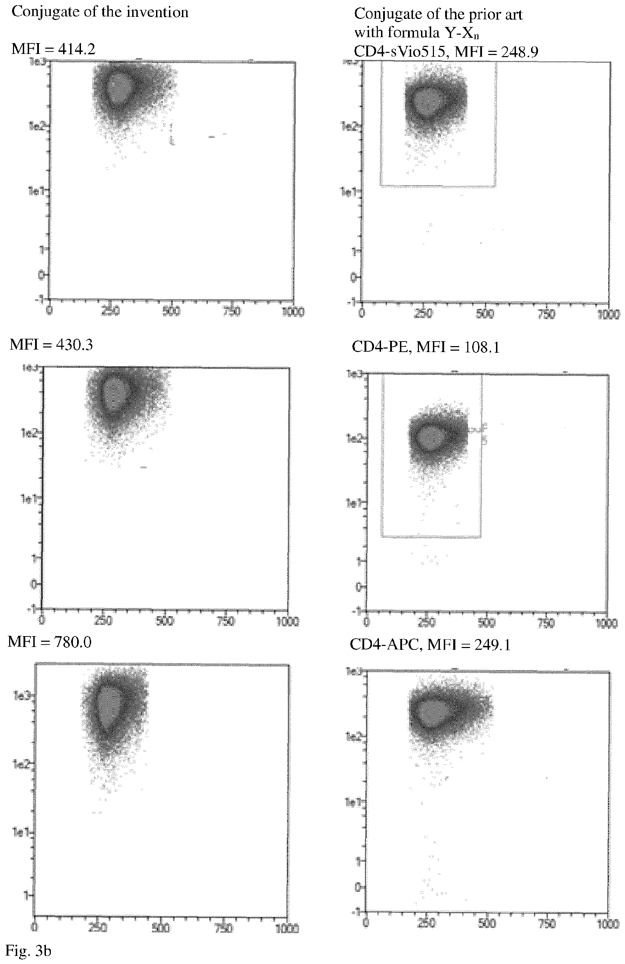

Fig. 3 : Flow cytometry dot plots of SUP-T1 cell stainings with dual-releasable conjugates. They show brighter stainings (measured in MFI) compared to their counterparts used in a regular cell staining setup

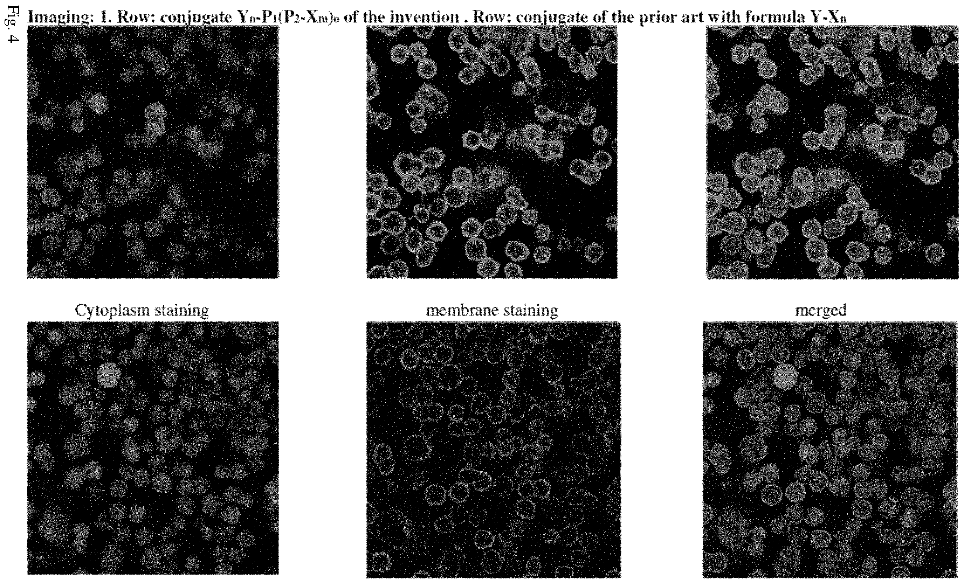

Fig. 4; Confocal laser scanning microscopy images of SUP-T1 cells stained with dual-releasable conjugates. It is demonstrated that they show brighter staining compared to regular cell staining conjugates.

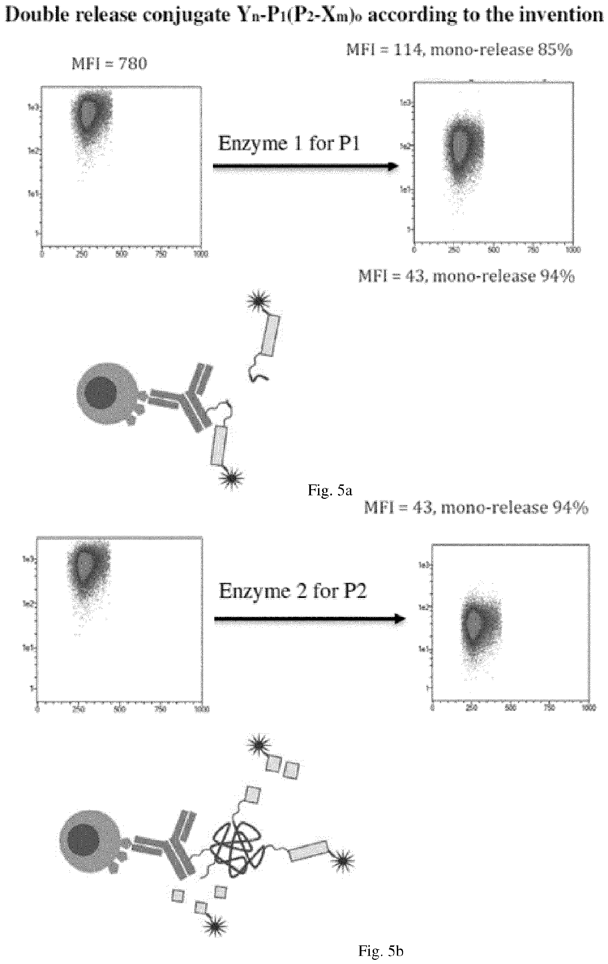

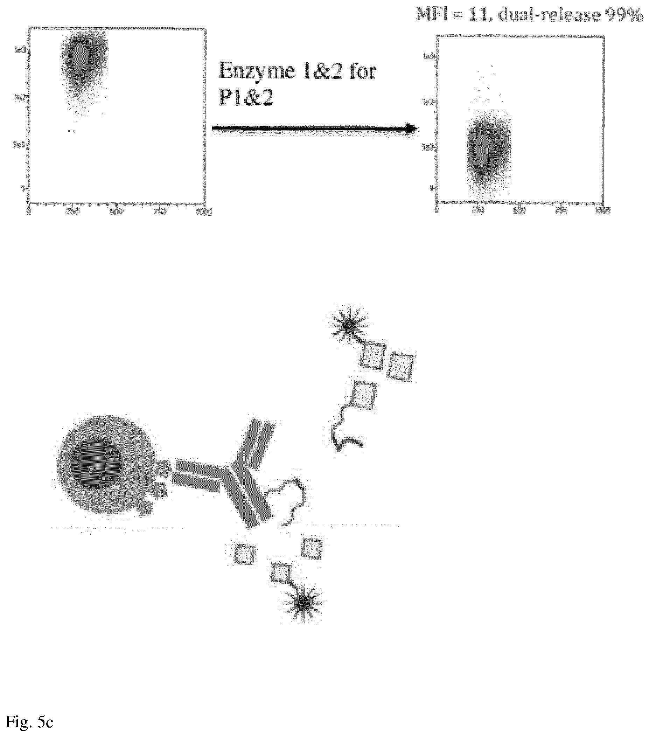

Fig. 5: Flow cytometry dot plots of SUP-T1 cell stainings with dual-releasable conjugates. It is demonstrated that simultaneous cleavage of P1 and P2 is more efficient than each cleavage by itself. It is also shown that cleavage can be performed orthogonally.

### DETAILED DESCRIPTION

Detection moiety X and antigen recognizing moiety Y can be bound covalently or quasi- covalently to the enzymatically degradable spacers P1 and P2. The terms "covalently or quasi- covalently" refers to bonds between X and P1; Y and P2; P1 and P2 having a dissociation constant of ≤ 10⁻⁹ M.

The term "cleaving the detection moiety X" means that the bond between X and P2 and/or Y and P1 is abrogated and detection moiety X may be removed from the target for example by washing.

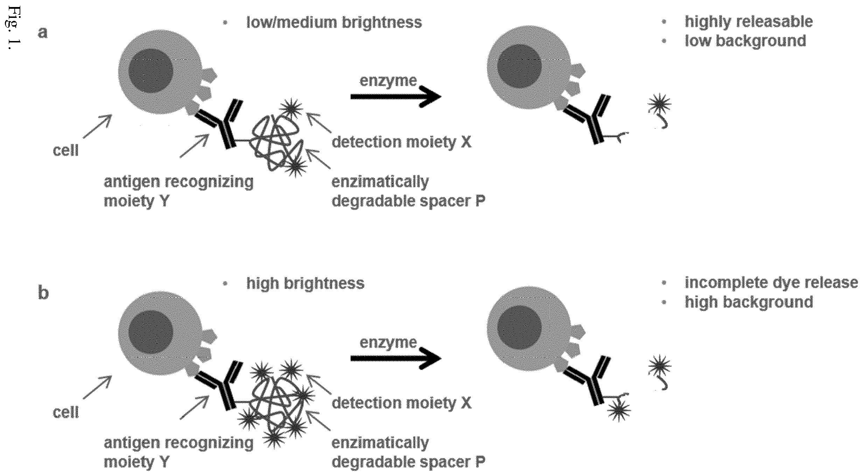

Fig. 1 shows schematically the method of the prior art by specific labeling of a target cell as biological specimen with conjugates having antigen recognizing moiety Y, enzymatically degradable spacers P1 and P2 and detection moiety X.

The process of the invention may be performed in one or more sequences of labeling and destaining. After each sequence, the detection moiety is released (removed) from the target moiety. Especially when the biological specimens are living cells which shall be further processed, the method of the invention has the advantage of providing unlabeled cells.

After each step of labeling and destaining a washing step can be performed to remove unwanted material like unbound conjugates or released detection moieties from the sample.

### Target moiety

The target moiety to be detected with the method of the invention can be on any biological specimen, like tissues slices, cell aggregates, suspension cells, or adherent cells. The cells may be living or dead. Preferable, target moieties are antigens expressed intracellular or extracellular on biological specimen like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens).

### Detection moiety

The detection moiety X of the conjugate may be any moiety possessing a property or function which can be used for detection purposes like those selected from the group consisting of chromophore moiety, fluorescent moiety, phosphorescent moiety, luminescent moiety, light absorbing moiety, radioactive moiety, and transition metal isotope mass tag moiety.

Suitable fluorescent moieties are those known from the field of fluorescence technologies, e.g., flow cytometry or fluorescence microscopy. In these embodiments of the invention, the target moiety labeled with the conjugate is detected by exciting the detection moiety X and detecting the resulting emission (photoluminescence). In this embodiment, the detection moiety X is preferable a fluorescent moiety.

Useful fluorescent moieties might be protein-based, such as phycobiliproteins, polymeric, such as polyfluorenes, small organic molecule dyes, such as xanthenes, like fluorescein, or rhodamines, cyanines, oxazines, coumarins, acridines, oxadiazoles, pyrenes, pyrromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

Another group of photoluminescent detection moieties are phosphorescent moieties with time-delayed emission of light after excitation. Phosphorescent moieties include metallo-organic complexes, such as Pd, Pt, Tb, Eu complexes, or nanoparticles with incorporated phosphorescent pigments such as lanthanide doped SrAl2O4.

In another embodiment of the invention the target labeled with the conjugate is detected without prior excitation by irradiation. In this embodiment the detection moiety can be a radioactive label. They may be in the form of radioisotope labeling by exchanging nonradioactive isotopes for their radioactive counterparts, such as tritium, 32P, 35S or 14C, or introducing covalently bound labels, such as 1251, which is bound to tyrosine, 18F within fluorodeoxyglucose, or metallo-organic complexes, i.e. 99Tc-DTPA.

In another embodiment the detection moiety is capable of causing chemiluminescence, i.e. horseradish peroxidase label in the presence of luminol.

In another embodiment of the invention the target labeled with the conjugate is not detected by radiation emission, but by absorption of UV, visible light, or NIR radiation. Suitable light-absorbing detection moieties are light absorbing dyes without fluorescence emission, such as small organic molecule quencher dyes like N-aryl rhodamines, azo dyes, and stilbenes.

In another embodiment, the light-absorbing detection moieties X can be irradiated by pulsed laser light, generating an photoacoustic signal.

In another embodiment of the invention the target labeled with the conjugate is detected by mass spectrometric detection of a transition metal isotope. Transition metal isotope mass tag labels might be introduced as covalently bound metallo-organic complexes or nanoparticle component. Known in the art are isotope tags of lanthanides and adjacent late transition elements.

The detection moiety X can be covalently or non-covalently coupled to the spacer P2. Methods for covalently or non-covalently conjugation are known by persons skilled in the art. In case of a covalent bound between the detection moiety X and the spacer P2, a direct reaction of an activated group either on the detection moiety or on the spacer P2 with an functional group on either the spacer P2 or on the detection moiety X or via an heterobifunctional linker molecule, which is firstly reacted with one and secondly reacted with the other binding partner is possible.

For example, a large number of heterobifunctional compounds are available for linking to entities. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-y-maleimidobutyryloxysuccinimide ester, N-hydroxy sulfosuccinimidyl-4- azidobenzoate, N-succinimidyl [4-azidophenyl]-1,3'-dithiopropionate, N-succinimidyl [4-iodoacetyl]aminobenzoate, glutaraldehyde, succinimidyl-[(N-maleimidopropionamido) polyethyleneglycol] esters (NHS-PEG-MAL), and succinimidyl 4-[N-maleimidomethyl]cyclohexane-1-carboxylate. A preferred linking group is 3-(2-pyridyldithio)propionic acid N-hydroxysuccinimide ester (SPDP), or 4-(N-maleimidomethyl)-cyclohexane-1-carboxylic acid N-hydroxysuccinimide ester (SMCC) with a reactive sulfhydryl group on the detection moiety and a reactive amino group on the spacers.

A quasi-covalent binding of the detection moiety X to the spacers can be achieved with binding systems providing a dissociation constant of ≤ 10⁻⁹ M, e.g., Biotin-Avidin binding interaction.

### Enzymatically degradable spacer P1 and P2

The enzymatically degradable spacers can be any molecule which can be cleaved by a specific enzyme, especially a hydrolase. Suitable as enzymatically degradable spacer P1 and P2 are, for example, polysaccharides, proteins, peptides, depsipeptides, polyesters, nucleic acids, and derivatives thereof.

Suitable polysaccharides are, for example, dextrans, pullulans, inulins, amylose, cellulose, hemicelluloses, such as xylan or glucomannan, pectin, chitosan, or chitin, which may be derivatized to provide functional groups for covalent or non-covalent binding of the detection moiety X and the antigen recognizing moiety Y. A variety of such modifications are known in the art, for example, imidazolyl carbamate groups may be introduced by reacting the polysaccharide with N,N'-carbonyl diimidazole. Subsequently amino groups may be introduced by reacting said imidazolyl carbamate groups with hexane diamine. Polysaccharides may also be oxidized using periodate to provide aldehyde groups or with N,N'-dicyclohexylcarbodiimide and dimethylsulfoxide to provide ketone groups. Aldehyde or ketone functional groups can be reacted subsequently preferably under conditions of reductive amination either with diamines to provide amino groups or directly with amino substituents on a proteinaceous binding moiety. Carboxymethyl groups may be introduced by treating the polysaccharide with chloroacetic acid. Activating the carboxy groups with methods known in the art which yield activated esters such N-hydroxysuccinimid ester or tetrafluorophenyl ester allows for reaction with amino groups either of a diamine to provide amino groups or directly with an amino group of a proteinaceous binding moiety. It is generally possible to introduce functional group bearing alkyl groups by treating polysaccharides with halogen compounds under alkaline conditions. For example, allyl groups can be introduced by using allyl bromide. Allyl groups can further be used in a thiol-ene reaction with thiol bearing compounds such as cysteamine to introduce amino groups or directly with a proteinaceous binding moiety with thiol groups liberated by reduction of disulfide bonds or introduced by thiolation for instance with 2-iminothiolane.

Proteins, peptides, and depsipeptides used as enzymatically degradable spacers can be functionalized via side chain functional groups of amino acids to attach detection moiety X and antigen recognizing moiety Y. Side chains functional groups suitable for modification are for instance amino groups provided by lysine or thiol groups provided by cysteine after reduction of disulfide bridges.

Polyesters and polyesteramides used as enzymatically degradable spacers can either be synthesized with co-monomers, which provide side chain functionality or be subsequently functionalized. In the case of branched polyesters functionalization can be via the carboxyl or hydroxyl end groups. Post polymerization functionalization of the polymer chain can be, for example, via addition to unsaturated bonds, i.e. thiolene reactions or azide-alkine reactions, or via introduction of functional groups by radical reactions.

Nucleic acids used as enzymatically degradable spacers are preferably synthesized with functional groups at the 3' and 5' termini suitable for attachment of the detection moiety X and antigen recognizing moiety Y. Suitable phosphoramidite building blocks for nucleic acid synthesis providing for instance amino or thiol functionalities are known in the art.

The enzymatically degradable spacers can be composed of more than one different enzymatically degradable units, which are degradable by the same or different enzyme.

### Antigen recognizing moiety Y

The term "antigen recognizing moiety Y" refers to any kind of antibody, nanobody, fragmented antibody or fragmented antibody derivatives, directed against the target moieties expressed on the biological specimens, like antigens expressed intracellular or extracellular on cells. The term relates to fully intact antibodies, fragmented antibody or fragmented antibody derivatives, e.g., Fab, Fab', F(ab')2, sdAb, scFv, di-scFv, nanobodies. Such fragmented antibody derivatives may be synthesized by recombinant procedures including covalent and non-covalent conjugates containing these kind of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules.

The conjugate used in the method of the invention may comprise up to 100, preferable 1- 10 antigen recognizing moieties Y. The interaction of the antigen recognizing moiety with the target antigen can be of high or low affinity. Binding interactions of a single low-affinity antigen recognizing moiety is too low to provide a stable bond with the antigen. Low-affinity antigen recognizing moieties can be multimerized by conjugation to the enzymatically degradable spacers to furnish high avidity. When the spacers are enzymatically cleaved in step d), the low-affinity antigen recognizing moieties will be monomerized which results in a complete removal of the detection moiety X, the spacers and the antigen recognizing moiety Y. High-affinity antigen recognizing moieties provide a stable bond which results in a removal of the detection moiety X and the spacers during step d).

Preferable, the term "Antigen recognizing moiety Y" refers to any antibody directed against antigen expressed by the biological specimens (target cells) intracellular, like IL2, FoxP3, CD154, or at the cell surface, like CD3, CD4, CD8, CD14, CD25, CD34, CD56, CD133, and EGFR.

The antigen recognizing moieties Y, especially antibodies, can be coupled to the spacer P through side chain amino or sufhydryl groups. In some cases the glycosidic side chain of the antibody can be oxidized by periodate resulting in aldehyde functional groups.

The antigen recognizing moiety Y can be covalently or non-covalently coupled to the spacer P. Methods for covalent or non-covalent conjugation are known by persons skilled in the art and the same as mentioned for conjugation of the detection moiety X.

The method of the invention is especially useful for detection and/or isolation of specific cell types from complex mixtures and may comprise more than one sequential or parallel sequences of statining and destaining. The method may use a variety of combinations of conjugates. For example, a conjugate may comprise antibodies specific for two different epitopes, like two different anti-CD34 antibodies. Different antigens may be addressed with different conjugates comprising different antibodies, for example, anti-CD4 and anti-CD8 for differentiation between two distinct T-cell-populations or anti-CD4 and anti-CD25 for determination of different cell subpopulations like regulatory T-cells.

### Enzymes

The choice of enzyme as release agent is determined by the chemical nature of the enzymatically degradable spacers P1 and P2 and can be one or a mixture of different enzymes. Enzymes are preferably hydrolases, but lyases or reductases are also possible. For example, if the spacer P1 or P2 is a polysaccharide, glycosidases (EC 3.2.1) are most suitable as release agents. Preferred are glycosidases that recognize specific glycosidic structures, e.g., dextranase (EC3.2.1.11), which cleaves at the (1->6) linkage of dextrans, pullulanases, which cleave either (1->6) linkages (EC 3.2.1.142) or (1->6) and (1->4) linkages (EC 3.2.1.41) of pullulans, neopullulanase (EC 3.2.1.135), and isopullulanase (EC 3.2.1.57), which cleave (1->4) linkages in pullulans. Amylase (EC 3.2.1.1), and maltogenic amylase (EC 3.2.1.133), which cleave (1->4) linkages in amylose, inulinase (EC 3.2.1.7), which cleaves β(2->1) fructosidic linkages in inulin, cellulase (EC 3.2.1.4), which cleaves at the (1->4) linkage of cellulose, xylanase (EC 3.2.1.8), which cleaves at the (1->4) linkages of xylan, pectinases such as endo-pectin lyase (EC 4.2.2.10), which cleaves eliminative at the (1->4) D-galacturonan methyl ester linkages, or polygalacturonase (EC 3.2.1.15), which cleaves at the (1->4) D-galactosiduronic linkages of pectin, chitosanase (EC 3.2.1.132), which cleaves at the (1->4) linkages of chitosan and endo-chitinase (EC 3.2.1.14) for cleaving of chitin.

Proteins and peptides may be cleaved by proteinases, which need to be sequence specific to avoid degradation of target structures on cells. Sequence specific proteases are for instance TEV protease (EC 3.4.22.44), which is a cysteine protease cleaving at the sequence ENLYFQ\S, enteropeptidase (EC 3.4.21.9), which is a serine protease cleaving after the sequence DDDDK, factor Xa (EC 3.4.21.6), which is a serine endopeptidase cleaving after the sequences IEGR or IDGR, or HRV3C protease (EC3.4.22.28), which is a cysteine protease cleaving at the sequence LEVLFQ\GP.

Depsipeptides, which are peptides containing ester bonds in the peptide backbone, or polyesters may be cleaved by esterases, such as porcine liver esterase (EC 3.1.1.1) or porcine pancreatic lipase (EC 3.1.1.3). Nucleic acids may be cleaved by endonucleases, which can be sequence specific, such as restriction enzymes (EC 3.1.21.3, EC 3.1.21.4, EC 3.1.21.5), such as *Eco*RI, *Hin*dII or *Bam*HI or more general such as DNAse I (EC 3.1.21.1), which cleaves phosphodiester linkages adjacent to a pyrimidine.

The amount of enzyme added needs to be sufficient to degrade substantially the spacer in the desired period of time. Usually the detection signal is at least about 80%, more usually at least about 95%, preferably at least about 99% reduced. The conditions for release may be empirically optimized in terms of temperature, pH, presence of metal cofactors, reducing agents, etc. The degradation will usually be completed in at least about 15 minutes, more usually at least about 10 minutes, and will usually not be longer than about 30 minutes.

### Cell Detection Methods

The method and equipment to detect the target labeled with the conjugate of the invention is determined by the detection moiety X.

In one variant of the invention the detection moiety X is a fluorescent moiety. Targets labeled with fluorochrome-conjugate are detected by exciting the fluorescent moiety X and analyzing the resulting fluorescence signal. The wavelength of the excitation is usually selected according to the absorption maximum of the fluorescent moiety X and provided by LASER or LED sources as known in the art. If several different detection moieties X are used for multiple colour/parameter detection, care should be taken to select fluorescent moieties having not overlapping absorption spectra, at least not overlapping absorption maxima. In case of a fluorescent moieties as detection moiety the targets may be detected, e.g., under a fluorescence microscope, in a flow cytometer, a spectrofluorometer, or a fluorescence scanner. Light emitted by chemiluminescence can be detected by similar instrumentation omitting the excitation.

In another variant of the invention the detection moiety is a light absorbing moiety, which is detected by the difference between the irradiation light intensity and the transmitted or reflected light intensity. Light absorbing moieties might also be detected by photoacoustic imaging, which uses the absorption of a pulsed laser beam to generate an acoustic like an ultrasonic signal.

Radioactive detection moieties are detected though the radiation emitted by the radioactive isotopes. Suitable instrumentation for detection of radioactive radiation include, for example, scintillation counters. In case of beta emission electron microscopy can also be used for detection.

Transition metal isotope mass tag moieties are detected by mass spectrometric methods such as ICP-MS, which is integrated in mass cytometry instrumentation.

### Use of the method

The method of the invention can be used for various applications in research, diagnostics and cell therapy.

In a first variant of the invention, biological specimens like cells are detected for counting purposes i.e. to establish the amount of cells from a sample having a certain set of antigens recognized by the antigen recognizing moieties of the conjugate.

In a second variant, one or more populations of biological specimens are detected from the sample and separated as target cells. This variant may be used for purification of target cells, for example, in clinical research, diagnostics, and immunotherapy. In this variant, one or more sorting steps may be performed after any of the staining and destaining steps and optionally washing step.

Suitable for such separations are especially flow sorters, e.g., FACS or MEMS-based cell sorter systems, for example as disclosed in EP14187215.0 or EP14187214.3.

In another variant of the invention, the location of the target moieties like antigens on the biological specimens recognized by the antigen recognizing moieties of the conjugate is determined. Such techniques are known as "Multi Epitope Ligand Cartography", "Chip-based Cytometry" or "Multioymx" and are described, for example, in EP 0810428, EP1181525, EP 1136822 or EP1224472. In this technology, cells are immobilized and contacted with antibodies coupled to fluorescent moiety. The antibodies are recognized by the respective antigens on the biological specimen (for example on a cell surface) and after removing the unbound marker and exciting the fluorescent moieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties.

The location of the target moieties is achieved by a digital imaging device with a sufficient resolution und sensitivity in for the wavelength of the fluorescence radiation. The digital imaging device may be used with or without optical enlargement for example with a fluorescence microscope. The resulting images are stored on an appropriate storing device like a hard drive, for example in RAW, TIF, JPEG, or HDF5 format.

In order to detect different antigens, different antibody-conjugates having the same or different fluorescent moiety or antigen recognizing moiety Y can be provided. Since the parallel detection of fluorescence emission with different wavelengths is limited, the antibody-fluorochrome-conjugates are utilized sequentially individually or in small groups (2-10) after the other.

In yet another variant of the method according to the invention, the biological specimens - especially suspension cells - of the sample are immobilized by trapping in microcavities or by adherence.

It is furthermore possible to provide next to conjugates of the invention with additional conjugates with the general formula (II) Xn -P'- Ym, with X, Y, n, m having the same meaning as in formula (I) and wherein X and Y are covalently or non-covalently bound to P' but with P' which can be a spacer which is not enzymatically degradable like a PEG spacer. In another variant, at least one non-enzymatically degradable conjugate with the general formula (III) Xn-Ym, wherein X, Y, n, m have the same meaning as in formula (I) can be provided. Non-enzymatically degradable conjugates with the general formula (II) and (III) survive the cleaving steps and can be used for further detection. Non-enzymatically degradable conjugates may be quenched by oxidative or radiation-induced destruction of the fluorescent moiety.

In general, the method of the invention can be performed in several variants. For example, the conjugate not recognized by a target moiety can be removed by washing for example with buffer before the target moiety labeled with the conjugate is detected.

In a variant of the invention, at least two conjugates are provided simultaneously or in subsequent staining sequences, wherein each antigen recognizing moiety Y recognizes different antigens. Here, the labeled target moieties can be detected simultaneously or sequentially. Sequential detection may involve simultaneous enzymatically degrading of the spacer molecules P1/P2 or subsequent enzymatically degrading of the spacer molecules P1/P2 with optionally intermediate removing (washing) of the non-bonded moieties.

Another variant of the invention comprises the elimination of a fluorescence emission by a combination of enzymatic degradation and oxidative bleaching. The necessary chemicals for bleaching are known from the above-mentioned publications on "Multi Epitope Ligand Cartography", "Chip-based Cytometry" or "Multioymx" technologies.

### EXAMPLES

### Preparation of conjugates:

### Step A: Preparation of reactive oligonucleotide (Oligo-Sulfhydryl)

The C6-S-S-Oligonucleotide was dissolved at a concentration of 2 µM in water and reduced with a 100-fold molar excess of a 100 mg/mL Tris-(2-carboxyethyl)-phosphin (TCEP) solution in water for 2 hours at room temperature (RT). Afterwards, it was purified from residual TCEP and C6-S protective group via size exclusion chromatography (SEC) under normal conditions.

### Step B: Preparation of reactive aminodextran (Dex-Maleimide)

A solution of aminodextran in phosphate buffered saline (PBS), pH 7.4, with a concentration of 20 mg/mL was incubated with a 50-fold molar excess of Succinimidyl 4-(N-maleimidomethyl)cyclohexane-1-carboxylate (SMCC) dissolved in dimethylsulfoxide (DMSO) at 20 mg/mL and incubated for 1 h at RT. Afterwards, it was purified from residual linker via SEC under normal conditions. The resulting compound had 28.7 maleimides available for conjugation.

### Step C: Preparation of oligonucleotide-dextran (Dex-Oligo)

Dex-Maleimide (4.5 mg/mL) as obtained in step B was incubated with Oligo-Sulfhydryl (2.4 mg/mL in PBS) as obtained in step A in a 40-fold molar excess for 1 h at RT and purified from unconjugated compounds via SEC under normal conditions.

### Step C: Preparation of reactive antibody (Ab-DBCO)

A solution of antibody in PBS (5.2 mg/mL) was treated with a solution of Dibenzocyclooctyne-N-hydroxysuccinimidyl ester (NHS-DBCO, 20 mg/mL) in DMSO in a 5-fold molar excess for 1 h at RT. Afterwards, it was purified from residual linker via SEC under normal conditions.

### Step D: Preparation of reactive oligonucleotide-dextran (N3-Dex-Oligo)

Dex-Oligo ( 1mg/mL) as obtained in step C was incubated with a solution of NHS azidoacetic acid (2 mg/mL) in DMSO in a 200-fold molar excess for 1 h at RT in PBS. Afterwards, it was purified from residual linker via SEC under normal conditions.

### Step E: Preparation of antibody-dextran-oligo conjugate (Ab-Dex-Oligo)

N3-Dex-Oligo (0.3 mg/mL) as obtained in step D was incubated with Ab-DBCO (10.3 mg/mL in PBS) as obtained in step C in a 50-fold molar excess for 2 h at RT and purified from unconjugated compounds via SEC under normal conditions. The resulting compound was comprised of 1.6 antibodies and 21.4 oligonucleotides per dextran.

### Step F: Preparation of biotin-functionalized antibody-dextran-oligo conjugate (Ab-Dex-dsOligo-Bio)

Complimentary biotinylated oligonucleotide (84 µM in water) was added to Ab-Dex-Oligo (250 nM) in a 43-fold excess in PBS and incubated for 10 min at 60 °C and cooled down to RT within 1 h under constant shaking. The crude was used in the next step without further purification.

### Step G: Preparation of staining reagent (Ab-Dex-dsOligo-Bio-aBiotin-Dye)

The crude Ab-Dex-dsOligo-Bio was incubated with aBiotin antibody dye conjugate in PBS at a 4-fold molar excess for 30 min prior to staining of cells in the dark.

### Step H: Cell staining

PBMCs in PBS/EDTA/BSA-buffer were stained for 10 min at 4 °C with the staining reagent as obtained in step G. The cells were washed with cold PBS/EDTA-BSA-buffer and analyzed by flow cytometry. For reversibility of the fluorescent labeling via dextran, cells were incubated with dextranase for 10 min at RT, washed with PBS/EDTA-BSA-buffer and analyzed by flow cytometry. For reversibility of the fluorescent labeling via DNA, cells were incubated with DNase I for 5 min at RT in 10 mM Hepes buffer at pH 7.4 containing 150mM NaCl, 5mM KCl, 1mM MgCl2, 1.8mM CaCl2, subsequently washed with PBS/EDTA-BSA-buffer and analyzed by flow cytometry. For reversibility of the fluorescent labeling via DNA and dextran, cells were incubated with DNase I and dextranase for 5 min at RT in 10 mM Hepes buffer at pH 7.4 containing 150mM NaCl, 5mM KCl, 1mM MgCl2, 1.8mM CaCl2, subsequently washed with PBS/EDTA-BSA-buffer and analyzed by flow cytometry.

## Claims

1. Conjugate with the general formula (I) Yₙ - P1(P2 - X ₘ)ₒ, with X: detection moiety; P1: first enzymatically degradable spacer; P2: second enzymatically degradable spacer; Y: antigen recognizing moiety and n, m, o integers between 1 and 100 with the provision that first spacer P1 and second spacer P2 are not degradable by the same enzyme.

2. Conjugate according to claim 1 **characterized in that** first spacer P1 and second spacer P2 are selected from the group consisting of polysaccharides, proteins, peptides, depsipeptides, polyesters, nucleic acids with the provisio that first spacer P1 and second spacer P2 are not selected from the same member of the group.

3. Conjugate according to claim 1 or 2 **characterized in that** the antigen recognizing moiety Y is an antibody, an fragmented antibody, an fragmented antibody derivative, peptide/MHC-complexes targeting TCR molecules, cell adhesion receptor molecules, receptors for costimulatory molecules or artificial engineered binding molecules.

4. Conjugate according to any of the claims 1 to 3 **characterized in that** the detection moiety is selected from the group consisting of chromophore moiety, fluorescent moiety, phosphorescent moiety, luminescent moiety, light absorbing moiety, radioactive moiety, transition metal and isotope mass tag moiety.

5. Method for detecting a target moiety in a sample of biological specimens **characterized in** :
a) providing at least one conjugate with the general formula (I) Yₙ - P1(P2 - Xₘ)ₒ, with X: detection moiety; P1: first enzymatically degradable spacer; P2: second enzymatically degradable spacer; and Y: antigen recognizing moiety and n, m, o are integers between 1 and 100
b) contacting the sample of biological specimens with at least one conjugate, thereby labeling the target moiety recognized by the antigen recognizing moiety Y
c) detecting the target moiety labeled with the conjugate with the detecting moiety X and
d) enzymatically degrading first spacer P1 and/or second P2 by providing a first enzyme capable of degrading first spacer P1 and/or a second enzyme capable of degrading second spacer enzyme P2, thereby cleaving the detection moiety X from the conjugate wherein the first enzyme is not capable of degrading second spacer P2 and the second enzyme is not capable of degrading first spacer P1.

6. Method according to claim 5 **characterized in that** by enzymatically degrading first and second spacers P1 and P2, the antigen recognizing moiety, Y is cleaved from the target moiety.

7. Method according to claim 5 or 6, wherein the enzyme used for degrading the first spacer P1 and the second spacer P2 are selected from the group consisting of glycosidases, dextranases, pullulanases, amylases, inulinases, cellulases, hemicellulases, pectinases, chitosanases, chitinases, proteinases, esterases, lipases, reductases, and nucleases with the provisio that the enzyme used for degrading the first spacer P1 and the second spacer P2 are not identical or not isozymes.

8. Method according to any of claims 5 to 7 **characterized by** contacting the sample of biological specimens with at least two conjugates having different detection moieties X and/or different enzymatically degradable spacers P and/or different antigen recognizing moieties Y in subsequent sequences comprising steps a) to d).

9. Method according to any of claims 5 to 7 **characterized by** contacting the sample of biological specimens in step b) simultaneously with at least two conjugates having different detection moieties X and/or different enzymatically degradable spacers P and/or different antigen recognizing moieties Y and performing the detection and cleaving of each conjugate in subsequent steps c) and d).

10. Method according to any of claims 5 to 7 **characterized by** contacting the sample of biological specimens in step b) simultaneously with at least two conjugates having different detection moieties X and/or different enzymatically degradable spacers P and/or different antigen recognizing moieties Y and simultaneous performing the detection and cleaving of each conjugate in step c) and d).

11. Method according to any of claims 5 to 7 **characterized by** contacting the sample of biological specimens with at least two conjugates having different detection moieties X and/or different enzymatically degradable spacers P and/or different antigen recognizing moieties Y in subsequent sequences comprising steps a) to c) and simultaneous performing the cleaving of the conjugates in a single step d)

12. Method according to any of claims 5 to 7 **characterized by** contacting the sample of biological specimens with at least two conjugates having different detection moieties X and/or different enzymatically degradable spacers P and/or different antigen recognizing moieties Y in subsequent sequences comprising steps a) to c) and performing the cleaving of each conjugate in subsequent steps d).

13. Method according to any of claims 5 to 7, **characterized by** contacting the sample of cells with at least two conjugates having different detection moieties X and/or different enzymatically degradable spacers P and/or different antigen recognizing moieties Y in subsequent sequences comprising steps a) to d) wherein step d) of the first conjugate and step b) of the second conjugate are performed simultaneously.
